# FASCICULE DE BREVET EUROPEEN

(11) **EP 1 366 750 B1**
(45) Date de publication et mention de la délivrance du brevet: **22.07.2009**
(21) Numéro de dépôt: 03291167.9
(22) Date de dépôt: 20.05.2003
(51) Int. Cl.: A61K 8/04, A61K 8/90, A61Q 5/06

(54) **Compositions capillaires préssurisées, comprenant au moins un copolymère linéaire diblocs amphiphile**
Druckhaarbehandlungsmittel enthaltend mindestens ein lineares, amphiphiles Diblock-Copolymer
Pressurised hair compositions comprising at least one linear amphiphilic diblock copolymer

(30) Priorité: 31.05.2002 FR 0206730
(43) Date de publication de la demande: 03.12.2003
(73) Titulaire: L'ORÉAL, 75008 Paris (FR)
(72) Inventeur: Dubief, Claude, 78150 Le Chesnay (FR); Giroud, Franck, 92110 Clichy (FR)
(74) Mandataire: Casalonga, Axel

(56) Documents cités:
- EP-A- 1 120 102
- WO-A-00/40628
- US-A- 3 907 984
- US-A- 4 261 972
- US-A- 5 019 377
- US-A- 5 104 642

## Description

La présente invention concerne des compositions capillaires, conditionnées dans un dispositif aérosol, comprenant dans un milieu cosmétiquement acceptable, au moins un copolymère linéaire séquencé diblocs, anionique ou non-ionique, constitué d'un bloc hydrophobe et d'un bloc hydrophile, et au moins un agent propulseur, ainsi que l'utilisation de ces compositions pour le coiffage des cheveux.

De nombreux polymères séquencés sont proposés actuellement pour la formulation de compositions destinées au coiffage, à la fixation et/ou au conditionnement des cheveux.
On peut citer, à titre d'exemples, les polymères de greffage à squelette hydrocarboné portant des greffons polydiméthylsiloxane (PDMS), les copolymères linéaires comportant des blocs à squelette hydrocarboné et des blocs PDMS, ou encore des polycondensats tels que les polyuréthannes à blocs polyester et/ou polyéther et/ou PDMS.
On connaît également des compositions de coiffage contenant des copolymères séquencés polyéthyléniques cationiques.
Ainsi, le brevet US 4 030 512 décrit des laques ou lotions de coiffage contenant des copolymères bi- ou tri-blocs cationiques comprenant au moins un bloc constitué de monomères aminés. Le caractère fortement cationique de ces copolymères rend difficile leur élimination par lavage et aboutit à une accumulation du polymère à la surface des cheveux. Il en résulte un toucher désagréable et artificiel après plusieurs applications.
Le brevet US 3 907 984 divulgue des compositions de fixation des cheveux contenant des copolymères biséquencés ou triséquencés, cationiques ou non ioniques, et qui sont insolubles dans l'eau mais solubles dans les solvants organiques tels que l'éthanol. Ces polymères, grâce à leur caractère hydrophobe, confèrent à la coiffure une bonne résistance à l'humidité. Les copolymères cationiques décrits dans ce document présentent les problèmes d'élimination au lavage évoqués ci-dessus, alors que les copolymères non-ioniques décrits donnent des performances cosmétiques médiocres. Ils présentent un faible pouvoir fixant et ont tendance à ternir les cheveux. Le brossage des cheveux traités avec ces copolymères se traduit par l'apparition d'un aspect poudreux qui contraint l'utilisateur à laver ses cheveux quotidiennement.

La demanderesse a constaté que l'utilisation, dans des compositions capillaires pressurisées, d'un groupe de copolymères séquencés particuliers, non ioniques ou anioniques, solubles ou dispersibles dans le milieu cosmétique utilisé, conduit à des formulations facilement pulvérisables, en particulier lorsqu'elles contiennent une forte proportion d'eau. Ces compositions s'appliquent aisément sur les cheveux et, de façon surprenante, conduisent beaucoup plus rapidement à l'aspect d'une chevelure sèche que les copolymères de l'art antérieur testés. Les copolymères séquencés non-ioniques ou anioniques présentent un pouvoir coiffant satisfaisant et s'éliminent facilement par un simple shampooing. Le brossage des cheveux traités ne conduit pas à un effet de poudrage mais laisse les cheveux doux et brillants.

La présente invention a par conséquent pour objet une composition capillaire, conditionnée dans un dispositif aérosol, comprenant, dans un milieu cosmétiquement acceptable,
- au moins un copolymère linéaire séquencé diblocs, anionique ou non ionique, constitué d'un bloc hydrophobe et d'un bloc hydrophile, à l'exclusion des copolymères séquencés d'oxyde d'éthylène et d'oxyde de propylène, des copolymères séquencés à motifs uréthanne et des copolymères séquencés à motifs siloxane, et
- au moins un agent propulseur.

Elle a en outre pour objet l'utilisation d'une telle composition capillaire pour le coiffage des cheveux.

Les copolymères linéaires séquencés diblocs, utilisables pour la présente invention, sont des copolymères dits "amphiphiles", à savoir des copolymères comportant à la fois un bloc hydrophobe et un bloc hydrophile.

On entend par "bloc hydrophobe" selon la présente invention un bloc comprenant au moins 75 % en moles de monomères insolubles dans l'eau et par "bloc hydrophile", un bloc comprenant au moins 75 % en moles de monomères hydrosolubles.

Les monomères hydrosolubles formant le bloc hydrophile des copolymères diblocs utilisés dans la présente invention peuvent être de nature anionique ou non-ionique et peuvent être utilisés seuls ou sous forme de mélange contenant deux ou plusieurs monomères différents.

On peut citer à titre d'exemples de monomères hydrosolubles anioniques, les acides carboxyliques à insaturation éthylénique, tels que l'acide acrylique, l'acide méthacrylique, l'acide itaconique, l'acide fumarique, l'acide crotonique et l'acide ou l'anhydride maléique, l'acide 2-acrylamido-2-méthylpropanesulfonique, l'acide styrènesulfonique, l'acide vinylsulfonique et l'acide vinylphosphonique.
Les monomères hydrosolubles non-ioniques englobent, entre autres, l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, l'acrylate d'hydroxypropyle, le méthacrylate d'hydroxyéthyle et le méthacrylate d'hydroxypropyle.

Les monomères insolubles dans l'eau formant le bloc hydrophobe des copolymères diblocs sont choisis de préférence parmi les monomères vinylaromatiques tels que le styrène et ses dérivés alkylés comme le 4-butylstyrène, 1'α-méthylstyrène et le vinyltoluène, les diènes tels que le butadiène et le 1,3-hexadiène, et les dérivés alkylés des diènes tels que l'isoprène et le diméthylbutadiène, le chloroprène, les acrylates d'alkyle en C_{1- 10}, d'aryle en C₆₋₁₀ ou d'aralkyle en C₆₋₁₀ et les méthacrylates d'alkyle en C₁₋₁₀, d'aryle en C₆₋₁₀ ou d'aralkyle en C₆₋₁₀, comme par exemple les (meth)acrylates de méthyle, d'éthyle, de n-butyle, de 2-éthylhexyle, de tert-butyle, d'isobornyle, de phényle ou de benzyle, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène, les monomères vinyliques fluorés ou à chaîne perfluorée, tels que les acrylates et méthacrylates de fluoroalkyle ou les α-fluoroacrylates d'alkyle.

Comme indiqué ci-dessus à propos de la définition des blocs hydrophobe et hydrophile des copolymères diblocs, les monomères insolubles dans l'eau et les monomères hydrosolubles représentent au moins 75 % en moles respectivement des blocs hydrophobe et hydrophile. Autrement dit, le bloc hydrophobe peut comprendre jusqu'à 25 % en moles d'un ou de plusieurs monomères hydrosolubles. Cette proportion est de préférence au plus égale 10 % en moles et, idéalement, inférieure ou égale à 5 % en moles.
De manière analogue, le bloc hydrophile peut comprendre jusqu'à 25 % en moles, de préférence jusqu'à 10 % en moles, et idéalement jusqu'à 5 % en moles, d'un ou de plusieurs monomères insolubles dans l'eau.
Les copolymères linéaires diblocs utilisés englobent bien entendu également ceux dans lesquels le bloc hydrophile et le bloc hydrophobe sont constitués exclusivement respectivement de monomères hydrosolubles et de monomères insolubles dans l'eau. Ces blocs peuvent être des blocs homopolymères ou des blocs copolymères renfermant deux ou plus de deux monomères différents du même type.

La masse moléculaire moyenne en nombre de chaque bloc, qu'il soit hydrophobe ou hydrophile, copolymère ou homopolymère, est de préférence comprise entre 500 et 100 000, en particulier entre 500 et 50 000, avec un indice de polydispersité (M_{w}/Mₙ) compris entre 1,01 et 3,0, de préférence entre 1,1 et 2,5.
Le rapport en poids du bloc hydrophobe au bloc hydrophile du copolymère séquencé est de préférence compris entre 1/20 et 20/1, et en particulier entre 1/10 et 10/1.

Les polymères diblocs de l'invention peuvent être préparés par les procédés de synthèse classiquement utilisés pour obtenir des polymères blocs. On peut citer par exemple les polymérisations anionique ou cationique, et la polymérisation radicalaire contrôlée (voir "New Method of Polymer Synthesis", Blackie Academic & Professional, Londres, 1995, volume 2, page 1, ou Trends Polym. Sci. 4, page 183 (1996) de C. J. Hawker), qui peut être mise en oeuvre suivant différents procédés comme par exemple la polymérisation radicalaire par transfert d'atomes *(Atom Transfert Radical Polymérisation* ou ATRP) (voir *JACS,* 117, page 5614 (1995), de Matyjasezwski *et al.),* la méthode des radicaux tels que les nitroxydes (Georges et al., Macromolecules, 1993, 26, 2987).
On peut aussi utiliser ces procédés pour obtenir un seul des deux types de blocs du polymère de l'invention, l'autre bloc étant introduit dans le polymère final par l'intermédiaire de l'amorceur utilisé ou bien par réaction de couplage entre les blocs hydrophiles et hydrophobes.

Les compositions capillaires de la présente invention contiennent de préférence les copolymères séquencés diblocs à l'état dissous ou finement dispersé dans le milieu cosmétiquement acceptable.
Les copolymères séquencés sont par conséquent de préférence solubles ou dispersibles dans le milieu cosmétiquement acceptable.

On entend au sens de la présente invention par "composés solubles" dans un milieu donné, des composés (monomères ou polymères) qui, introduits dans ledit milieu à 25°C, à une concentration en poids égale à 0,5 %, si besoin est, neutralisés, permettent l'obtention d'une solution macroscopiquement homogène et transparente, c'est-à-dire ayant une valeur de transmittance de la lumière, à une longueur d'onde égale à 500 nm, à travers un échantillon de 1 cm d'épaisseur, d'au moins 70 %, de préférence d'au moins 80 %.
On entend par "composés dispersibles" dans un milieu, des composés (monomères ou polymères) qui, introduits dans ledit milieu à 25 °C à une concentration en poids égale à 0,5 % en poids permettent l'obtention d'une dispersion homogène non transparente.

On entend par milieu cosmétiquement acceptable selon l'invention un milieu constitué d'eau ou d'un ou plusieurs solvants organiques cosmétiquement acceptables, tels que par exemple les alcools inférieurs en C₁₋₄ en particulier l'éthanol, l'isopropanol, le tertio-butanol et le n-butanol, et les alkylèneglycols comme le propylèneglycol, ou encore un mélange d'eau et d'un ou de plusieurs solvants organiques cosmétiquement acceptables.
La composition du milieu cosmétiquement acceptable et sa proportion dans la composition capillaire finale sont de préférence telles que la teneur globale de la composition capillaire en composés organiques volatils (VOC, *volatil organic compounds)* est au plus égale à 55 % en poids.

La quantité de copolymère linéaire diblocs amphiphile dans les compositions capillaires de la présente invention dépend d'un grand nombre de paramètres parmi lesquels on peut citer la masse moléculaire des copolymères diblocs, les tailles respectives du bloc hydrophile et hydrophobe, et en particulier de l'effet cosmétique recherché.
On obtient généralement un pouvoir coiffant satisfaisant avec une quantité de copolymères linéaires diblocs comprise entre 0,01 et 20 % en poids, de préférence entre 0,1 et 15 % en poids rapporté au poids total de la composition capillaire.
On entend par pouvoir coiffant l'aptitude d'une substance à faciliter la mise en forme de la chevelure et à maintenir cette forme pendant une durée plus ou moins longue.

L'agent propulseur peut être n'importe quel gaz liquéfiable utilisé habituellement dans des dispositifs aérosol, tels que le diméthyléther, les alcanes en C₃₋₅, le 1,1-difluoroéthane, les mélanges de diméthyléther et d'alcanes en C₃₋₅, et les mélanges de 1,1-difluoroéthane et de diméthyléther et/ou d'alcanes en C₃₋₅.

On préfère tout particulièrement utiliser comme agent propulseur le diméthyléther et les alcanes en C₃₋₅, et en particulier le propane, le n-butane et l'isobutane.

Le rapport pondéral phase liquide/agent propulseur des compositions capillaires pressurisées de la présente invention est de préférence compris entre 50 et 0,05, et en particulier entre 25 et 1,5.

Les compositions capillaires de la présente invention peuvent contenir en outre un ou plusieurs ingrédients cosmétiques ou de formulation, utilisés habituellement dans le domaine cosmétique.
On peut citer à titre d'exemples de tels ingrédients les polymères filmogènes anioniques, cationiques, non-ioniques ou amphotères, différents des copolymères diblocs amphiphiles décrits ci-dessus, les silicones volatiles ou non volatiles, les agents tensioactifs anionique, cationiques, amphotères ou non-ioniques, les agents épaississants, les agents nacrants, les filtres UV, les agents anti-radicalaires, les parfums, les agents conservateurs, les pigments et colorants, les agents d'ajustement du pH, les agents solubilisants, les agents plastifiants, les agents anti-mousse, les cires et huiles, les vitamines, des agents conditionneurs et les particules organiques ou minérales, synthétiques ou d'origine naturelle.
Bien entendu l'homme du métier veillera à choisir ce ou ces éventuels additifs complémentaires et/ou leurs quantités de manière à ce que les propriétés avantageuses intrinsèques des compositions de l'invention ne soient pas altérées par la ou les adjonctions envisagées.

Les compositions capillaires sont de préférence des laques pour cheveux ou des mousses.

L'invention est illustrée à l'aide de l'exemple suivant.

### Exemple 1

### Laques capillaires

On prépare cinq laques capillaires (Compositions A à E) ayant une teneur en composés organiques volatils de 55 % en poids, et contenant chacune respectivement 10 % en poids de copolymères fixants séquencés.

Les compositions A et B selon l'invention contiennent respectivement des copolymères diblocs anioniques et non-ioniques, alors que les compositions comparatives C et D contiennent des copolymères séquencés cationiques et la composition comparative E un polyuréthanne multiblocs.

| | A | B | C | D | E |
|---|---|---|---|---|---|
| dibloc anionique PS-b-PAA^{(a)} | 10 % | | | | |
| dibloc non-ionique PS-b-PHEMA^{(b)} | | 10 % | | | |
| dibloc cationique^{(c)} | | | 10 % | | |
| dibloc cationique^{(d)} | | | | 10 % | |
| polyuréthane multiblocs^{(e)} | | | | | 10 % |
| parfum | q.s. | q.s. | q.s. | q.s. | q.s. |
| éthanol | 20% | 20% | 20% | 20 % | 20% |
| AMP | pH7 | pH7 | pH7 | pH7 | pH7 |
| DME | 35 % | 35 % | 35% | 35 % | 35 % |
| eau | qsp 100% | qsp 100% | qsp 100% | qsp 100% | qsp 100% |

| | | | | | |
|---|---|---|---|---|---|
| (a) diblocs polystyrène(1500g/mole)-b-poly(acide acrylique)(44000g/mole) commercialisé par la société Polymer Source, Inc. (b) diblocs polystyrène(1500 g/mole)-b-poly(méthacrylate d'hydroxyéthyle)(40000 g/mole) commercialisé par la société Polymer Source, Inc. (c) dibloc polystyrène(18600g/mole)-poly(iodure de N-méthyl-4-vinylpyridinium)(131300 g/mole) commercialisé par la société Polymer Source, Inc. (d) diblocs poly(méthacrylate de lauryle-b-méthacrylate de N,N-diméthylaminoéthyle), polymère diblocs cationique selon US 4 030 512 (e) LUVISET^{®} PUR, polyuréthane multiblocs anionique commercialisé par la société BASF | | | | | |

Chacune des compositions ci-dessus est appliquée à l'aide d'un dispositif aérosol sur 10 chevelures de cheveux européens de couleur châtain mi-longs (20 cm environ) à raison de 10 grammes par chevelure.
Un groupe de 5 experts évalue la finesse de la nébulisation, la vitesse de séchage, le pouvoir laquant et la facilité d'élimination par lavage à l'aide d'une échelle de notation allant de 0 à 5.

Les résultats, exprimés sous forme de moyenne des notes attribuées, sont résumés dans le tableau ci-dessous.

| | **A selon l'invention** | **B selon l'invention** | C | D | E |
|---|---|---|---|---|---|
| qualité de la vaporisation | 4,3 | 3,8 | 3,7 | 4,1 | **1,9** |
| vitesse de séchage | 4,8 | 3,5 | 3,5 | 3,2 | **1,0** |
| pouvoir laquant | 3,6 | 3,9 | 4,1 | 4,3 | 3,5 |
| élimination par lavage | 4,5 | 4,8 | **0,5** | **1,1** | 3,6 |

Ces résultats montrent que les polymères diblocs cationiques utilisés dans l'état de la technique donnent des résultats cosmétiques comparables aux polymères diblocs anioniques ou non-ioniques selon l'invention mais présentent l'inconvénient d'être difficiles à éliminer par lavage, ce qui se traduit par un toucher chargé, peu naturel après lavage.
Les résultats montrent en outre que les compositions cosmétiques selon l'invention sèchent plus rapidement et permettent une meilleure pulvérisation qu'une composition de l'état de la technique (composition E) contenant un polyuréthane multiblocs amphiphile anionique.

## Revendications

1. Composition capillaire, conditionnée dans un dispositif aérosol, comprenant, dans un milieu cosmétiquement acceptable,
• au moins un copolymère linéaire séquencé diblocs, anionique ou non ionique, constitué d'un bloc hydrophobe et d'un bloc hydrophile, à l'exclusion des copolymères séquencés d'oxyde d'éthylène et d'oxyde de propylène, des copolymères séquencés à motifs uréthanne et des copolymères séquencés à motifs siloxane, et
• au moins un agent propulseur.

2. Composition capillaire selon la revendication 1, **caractérisée par le fait que** le ou les copolymères linéaires séquencés diblocs sont solubles ou dispersibles dans le milieu utilisé.

3. Composition capillaire selon la revendication 1 ou 2, **caractérisée par le fait que** le bloc hydrophile est formé de monomères hydrosolubles choisis parmi les monomères hydrosolubles anioniques, les monomères hydrosolubles non-ioniques ou un mélange de ceux-ci.

4. Composition capillaire selon la revendication 3, **caractérisée par le fait que** les monomères hydrosolubles anioniques sont choisis parmi les acides carboxyliques à insaturation éthylénique, l'acide 2-acrylamido-2-méthylpropanesulfonique, l'acide styrènesulfonique, l'acide vinylsulfonique et l'acide vinylphosphonique.

5. Composition capillaire selon la revendication 3, **caractérisée par le fait que** les monomères hydrosolubles non-ioniques sont choisis parmi l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique, l'oxyde d'éthylène, l'acrylate d'hydroxyéthyle, l'acrylate d'hydroxypropyle, le méthacrylate d'hydroxyéthyle et le méthacrylate d'hydroxypropyle.

6. Composition capillaire selon l'une quelconque des revendications précédentes, **caractérisée par le fait que** le bloc hydrophobe est formé de monomères insolubles dans l'eau choisis parmi les monomères vinylaromatiques, les diènes et les dérivés alkylés des diènes, le chloroprène, les acrylates d'alkyle en C₁₋₁₀, d'aryle en C₆₋₁₀ ou d'aralkyle en C₁₋₁₀, les méthacrylates d'alkyle en C₁₋₁₀, d'aryle en C₆₋₁₀ ou d'aralkyle en C₁₋₁₀, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène et les monomères vinyliques fluorés ou à chaîne perfluorée.

7. Composition capillaire selon l'une quelconque des revendications précédentes, **caractérisée par le fait que** le bloc hydrophile contient jusqu'à 25 % en moles, de préférence jusqu'à 10 % en moles, et idéalement jusqu'à 5 % en moles, d'un ou de plusieurs monomères insolubles dans l'eau selon la revendication 6.

8. Composition capillaire selon l'une quelconque des revendications précédentes, **caractérisée par le fait que** le bloc hydrophobe contient jusqu'à 25 % en moles, de préférence jusqu'à 10 % en moles, et idéalement jusqu'à 5 % en moles, d'un ou de plusieurs monomères hydrosolubles selon l'une quelconque des revendications 3 à 5.

9. Composition capillaire selon l'une quelconque des revendications précédentes, **caractérisée par le fait que** le rapport en poids du bloc hydrophobe au bloc hydrophile du copolymère dibloc est compris entre 1/20 et 20/1, de préférence entre 1/10 et 10/1.

10. Composition capillaire selon l'une quelconque des revendications précédentes, **caractérisée par le fait que** le ou les copolymères linéaires diblocs sont présents à raison de 0,01 à 20 % en poids, de préférence à raison de 0,1 à 15 % en poids, rapporté au poids total de la composition.

11. Composition capillaire selon l'une quelconque des revendications précédentes, **caractérisée par le fait que** l'agent propulseur est choisi parmi le diméthyléther, les alcanes en C₃₋₅, le 1,1-difluoroéthane, les mélanges de diméthyléther et d'alcanes en C₃₋₅, et les mélanges de 1,1-difluoroéthane et de diméthyléther et/ou d'alcanes en C₃₋₅.

12. Composition capillaire selon l'une quelconque des revendications précédentes, **caractérisée par le fait qu'**elle contient en outre des additifs cosmétiques et/ou des adjuvants de formulation tels que les polymères filmogènes anioniques, cationiques, non-ioniques ou amphotères, solubles ou dispersibles dans un milieu aqueux ou hydroalcoolique, les silicones volatiles ou non volatiles, les agents tensioactifs anionique, cationiques, amphotères ou non-ioniques, les agents épaississants, les agents nacrants, les filtres UV, les agents anti-radicalaires, les parfums, les agents conservateurs, les pigments et colorants, les agents d'ajustement du pH, les agents solubilisants, les agents plastifiants, les agents anti-mousse, les cires et huiles, les vitamines, des agents conditionneurs et les particules organiques ou minérales, synthétiques ou d'origine naturelle.

13. Composition capillaire selon l'une quelconque des revendications précédentes, **caractérisée par le fait qu'**elle contient au plus 55 % en poids de composés organiques volatils.

14. Utilisation d'une composition capillaire selon l'une quelconque des revendications précédentes, pour le coiffage des cheveux.

## Claims

1. Hair composition, packaged in an aerosol device, comprising, in a cosmetically acceptable medium,
• at least one anionic or nonionic linear diblock block copolymer, consisting of a hydrophobic block and a hydrophilic block, with the exclusion of block copolymers of ethylene oxide and of propylene oxide, block copolymers containing urethane units and block copolymers containing siloxane units, and
• at least one propellant.

2. Hair composition according to Claim 1, **characterized in that** the linear diblock block copolymer(s) are soluble or dispersible in the medium used.

3. Hair composition according to Claim 1 or 2, **characterized in that** the hydrophilic block is formed from water-soluble monomers chosen from anionic water-soluble monomers, nonionic water-soluble monomers or a mixture thereof.

4. Hair composition according to Claim 3, **characterized in that** the anionic water-soluble monomers are chosen from ethylenically unsaturated carboxylic acids, 2-acrylamido-2-methylpropanesulphonic acid, styrenesulphonic acid, vinylsulphonic acid and vinylphosphonic acid.

5. Hair composition according to Claim 3, **characterized in that** the nonionic water-soluble monomers are chosen from acrylamide, C₁₋₆ N-alkyl or C₁₋₃ N,N-dialkyl acrylamides, polyethylene glycol acrylate, polyethylene glycol methacrylate, N-vinylacetamide, N-methyl-N-vinylacetamide, N-vinylformamide, N-methyl-N-vinylformamide, N-vinyllactams comprising a cyclic group of 4 to 9 carbon atoms, vinyl alcohol, ethylene oxide, hydroxyethyl acrylate, hydroxypropyl acrylate, hydroxyethyl methacrylate and hydroxypropyl methacrylate.

6. Hair composition according to any one of the preceding claims, **characterized in that** the hydrophobic block is formed from water-insoluble monomers chosen from vinylaromatic monomers, dienes and alkyl derivatives of dienes, chloroprene, C₁₋₁₀ alkyl, C₆₋₁₀ aryl or C₁₋₁₀ aralkyl acrylates, C₁₋₁₀ alkyl, C₆₋₁₀ aryl or C₁₋₁₀ aralkyl methacrylates, vinyl acetate, the vinyl ethers of formula CH₂=CH-O-R and the allyl ethers of formula CH₂=CH-CH₂-O-R in which R represents a C₁₋₆ alkyl group, acrylonitrile, vinyl chloride, vinylidene chloride, caprolactone, ethylene, propylene and fluorovinyl monomers or vinyl monomers containing a perfluoro chain.

7. Hair composition according to any one of the preceding claims, **characterized in that** the hydrophilic block contains up to 25 mol%, preferably up to 10 mol% and ideally up to 5 mol% of one or more water-insoluble monomers according to Claim 6.

8. Hair composition according to any one of the preceding claims, **characterized in that** the hydrophobic block contains up to 25 mol%, preferably up to 10 mol% and ideally up to 5 mol% of one or more water-soluble monomers according to any one of Claims 3 to 5.

9. Hair composition according to any one of the preceding claims, **characterized in that** the weight ratio of the hydrophobic block to the hydrophilic block in the diblock copolymer is between 1/20 and 20/1 and preferably between 1/10 and 10/1.

10. Hair composition according to any one of the preceding claims, **characterized in that** the linear diblock copolymer(s) is(are) present in a proportion of from 0.01% to 20% by weight and preferably in a proportion of from 0.1% to 15% by weight, relative to the total weight of the composition.

11. Hair composition according to any one of the preceding claims, **characterized in that** the propellant is chosen from dimethyl ether, C₃₋₅ alkanes, 1,1-difluoroethane, mixtures of dimethyl ether and of C₃₋₅ alkanes, and mixtures of 1,1-difluoroethane and of dimethyl ether and/or of C₃₋₅ alkanes.

12. Hair composition according to any one of the preceding claims, **characterized in that** it also contains cosmetic additives and/or formulation adjuvants such as anionic, cationic, nonionic or amphoteric film-forming polymers that are soluble or dispersible in an aqueous or aqueous-alcoholic medium, volatile or non-volatile silicones, anionic, cationic, amphoteric or nonionic surfactants, thickeners, nacreous agents, UV-screening agents, free-radical scavengers, fragrances, preserving agents, pigments, colorants, pH regulators, solubilizers, plasticizers, antifoams, waxes, oils, vitamins, conditioners and organic or mineral particles, which are synthetic or of natural origin.

13. Hair composition according to any one of the preceding claims, **characterized in that** it contains not more than 55% by weight of volatile organic compounds.

14. Use of a hair composition according to any one of the preceding claims, for styling the hair.

## Patentansprüche

1. Zusammensetzung für die Haarbehandlung, die in einem Aerosolbehälter konfektioniert ist und die in einem kosmetisch akzeptablen Medium enthält:
- mindestens ein anionisches oder nichtionisches lineares Zweiblock-Sequenzcopolymer, das aus einem hydrophoben Block und einem hydrophilen Block besteht, wobei Sequenzcopolymere von Ethylenoxid und Propylenoxid, Sequenzcopolymere mit Urethaneinheiten und Sequenzcopolymere mit Siloxaneinheiten ausgenommen sind, und
- mindestens ein Treibmittel.

2. Zusammensetzung für die Haarbehandlung nach Anspruch 1, **dadurch gekennzeichnet, dass** das oder die linearen Zweiblock-Sequenzcopolymere in dem verwendeten Medium löslich oder dispergierbar sind.

3. Zusammensetzung für die Haarbehandlung nach Anspruch 1 oder 2, **dadurch gekennzeichnet, dass** der hydrophile Block aus wasserlöslichen Monomeren gebildet ist, die unter den wasserlöslichen anionischen Monomeren, wasserlöslichen nichtionischen Monomeren oder einem Gemisch aus diesen Monomeren ausgewählt sind.

4. Zusammensetzung für die Haarbehandlung nach Anspruch 3, **dadurch gekennzeichnet, dass** die anionischen wasserlöslichen Monomere unter den Carbonsäuren mit ethylenisch ungesättigter Bindung, 2-Acrylamido-2-methylpropansulfonsäure, Styrolsulfonsäure, Vinylsulfonsäure und Vinylphosphonsäure ausgewählt sind.

5. Zusammensetzung für die Haarbehandlung nach Anspruch 3, **dadurch gekennzeichnet, dass** die nichtionischen wasserlöslichen Monomere unter Acrylamid, N-Alkyl(C₁₋₆)acrylamiden oder N,N-Dialkyl(C₁₋₃)acrylamiden, Polyethylenglycolacrylat, Polyethylenglycolmethacrylat, N-Vinylacetamid, N-Methyl-N-vinylacetamid, N-Vinylformamid, N-Methyl-N-vinylformamid, N-Vinyllactamen, die eine cyclische Gruppe mit 4 bis 9 Kohlenstoffatomen aufweisen, Vinylalkohol, Ethylenoxid, Hydroxyethylacrylat, Hydroxypropylacrylat, Hydroxyethylmethacrylat und Hydroxypropylmethacrylat ausgewählt sind.

6. Zusammensetzung für die Haarbehandlung nach einem der vorhergehenden Ansprüche, **dadurch gekennzeichnet, dass** der hydrophobe Block aus in Wasser unlöslichen Monomeren gebildet ist, die unter den vinylaromatischen Monomeren, Dienen und alkylierten Dienderivaten, Chloropren, Alkyl(C₁₋₁₀)acrylaten, Aryl(C₆₋₁₀)acrylaten oder Aralkyl(C₁₋₁₀)acrylaten, Alkyl(C₁₋₁₀)methacrylaten, Aryl(C₆₋₁₀)methacrylaten oder Aralkyl(C₁₋₁₀)methacrylaten, Vinylacetat, Vinylethern der Formel CH₂=CH-O-R und Allylethern der Formel CH₂=CH-CH₂-O-R, worin R eine C₁₋₆-Alkylgruppe bedeutet, Acrylnitril, Vinylchlorid, Vinylidenchlorid, Caprolacton, Ethylen, Propylen und fluorierten Vinylmonomeren oder Vinylmonomeren mit perfluorierter Kette ausgewählt sind.

7. Zusammensetzung für die Haarbehandlung nach einem der vorhergehenden Ansprüche, **dadurch gekennzeichnet, dass** der hydrophile Block bis zu 25 Mol-%, vorzugsweise bis zu 10 Mol-% und idealerweise bis zu 5 Mol-% eines oder mehrerer in Wasser unlöslicher Monomere nach Anspruch 6 enthält.

8. Zusammensetzung für die Haarbehandlung nach einem der vorhergehenden Ansprüche, **dadurch gekennzeichnet, dass** der hydrophobe Block bis zu 25 Mol-%, vorzugsweise bis zu 10 Mol-% und idealerweise bis zu 5 Mol-% eines oder mehrerer wasserlöslicher Monomere nach einem der Ansprüche 3 bis 5 enthält.

9. Zusammensetzung für die Haarbehandlung nach einem der vorhergehenden Ansprüche, **dadurch gekennzeichnet, dass** das Gewichtsverhältnis des hydrophoben Blocks und des hydrophilen Blocks des Zweiblock-Copolymers im Bereich von 1/20 bis 20/1 und vorzugsweise 1/10 bis 10/ 1 liegt

10. Zusammensetzung für die Haarbehandlung nach einem der vorhergehenden Ansprüche, **dadurch gekennzeichnet, dass** das oder die linearen Zweiblock-Sequenzcopolymere in einer Menge von 0,01 bis 20 Gew.-% und vorzugsweise 0,1 bis 15 Gew.-%, bezogen auf das Gesamtgewicht die Zusammensetzung, enthalten sind.

11. Zusammensetzung für die Haarbehandlung nach einem der vorhergehenden Ansprüche, **dadurch gekennzeichnet, dass** das Treibmittel unter Dimethylether, C₃₋₅-Alkanen, 1,1-Difluorethan, Gemischen von Dimethylether und C₃₋₅-Alkanen und Gemischen von 1,1-Difluorethan und Dimethylether und/oder C₃₋₅-Alkanen ausgewählt ist.

12. Zusammensetzung für die Haarbehandlung nach einem der vorhergehenden Ansprüche, **dadurch gekennzeichnet, dass** sie ferner kosmetische Zusatzstoffe und/oder Formulierungshilfsstoffe enthält, wie anionische, kationische, nichtionische oder amphotere filmbildende Polymere, die in einem wässrigen oder wässrigalkoholischen Medium löslich oder dispergierbar sind, flüchtige oder nicht flüchtige Silicone, anionische, kationische, amphotere oder nichtionische grenzflächenaktive Stoffe, Verdickungsmittel, Perlglanzstoffe, UV-Filter, Radikalfänger für freie Radikale, Parfums, Konservierungsmittel, Pigmente und Farbstoffe, pH-Regler, Solubilisierungsmittel, Weichmacher, Schaumverhütungsmittel, Wachse und Öle, Vitamine, Konditioniermittel und organische oder anorganische, synthetische Partikel oder organische oder anorganische Partikel natürlicher Herkunft.

13. Zusammensetzung für die Haarbehandlung nach einem der vorhergehenden Ansprüche, **dadurch gekennzeichnet, dass** sie höchstens 55 Gew.-% flüchtige organische Verbindungen enthält.

14. Verwendung einer Zusammensetzung für die Haarbehandlung nach einem der vorhergehenden Ansprüche für die Frisurengestaltung.
